# EUROPEAN PATENT APPLICATION

(11) **EP 4 837 895 A1**
(43) Date of publication of application: **11.11.2026**
(21) Application number: 25766469.8
(22) Date of filing: 26.02.2025
(51) Int. Cl.: C07B 63/00, A61K 31/53, B01D 9/00

(54) **PROCESS AND DEVICE FOR PRODUCING SPECIALTY MELAMINE USING GAS-PHASE QUENCHING METHOD**

(30) Priority: 19.02.2025 CN 202510184085
(71) Applicant: Sichuan Golden-Elephant Sincerity Chemical Co., Ltd, Meishan, Sichuan 620031 (CN); Beijing Edgein Technology Co., Ltd, Beijing 100036 (CN)
(72) Inventor: TANG, Yin, 620031 Meishan, Sichuan (CN); KONG, Deli, 620031 Meishan, Sichuan (CA); SONG, Guotian, 620031 Meishan, Sichuan (CN); MO, Yuxin, 620031 Meishan, Sichuan (CN); CAO, Xuegang, 620031 Meishan, Sichuan (CN); LI, Jiadong, 620031 Meishan, Sichuan (CN); QIN, Yanni, 620031 Meishan, Sichuan (CN); LI, Zhonggyun, 620031 Meishan, Sichuan (CN)
(74) Representative: Lavoix
(86) International application number: PCT/CN2025/079331
(87) International publication number: WO 2026/174609

(57) **Abstract**

The present application discloses a process and apparatus for producing specialty melamine by gas-phase quenching. The production process comprises: admitting hot process gas from the outlet of the melamine reactor to contact cold process gas in a crystallizer, thereby producing solid A and mixed gas, discharging the solid A from the bottom of the crystallizer, and feeding the mixed gas into a separator to perform gas-solid separation and thereby obtaining solid B, wherein the solid A is transported into a high-pressure collecting device A using high-pressure conveying gas A, a temperature of high-pressure conveying gas A is ensured to be 120°C or above during transportation; after performing gas-solid separation in the high-pressure collecting device A, the solid A is transported into a packaging device A using low-pressure conveying gas A, and a temperature of solid A at the end of transportation pipe is ensured not to exceed 85°C.

## Description

### CROSS-REFERENCE TO RELATED APPLICATION

The present application claims priority to the Chinese Patent Application No. 202510184085.1, filed to the China National Intellectual Property Administration on February 19, 2025, entitled "PROCESS AND APPARATUS FOR PRODUCING SPECIALTY MELAMINE BY GAS-PHASE QUENCHING", and the entire contents of which are incorporated herein by reference.

### TECHNICAL FIELD

The present application relates to the field of melamine production, specifically to a process and apparatus for producing specialty melamine by gas-phase quenching.

### BACKGROUND

Melamine is a widely used basic organic chemical intermediate product. Its primary application is as a raw material for the production of melamine-formaldehyde resin (MF). In addition, melamine can also be used in wood processing, flame retardants, fiber reinforcing agent, water reducing agent, formaldehyde scavenger. Melamine is usually produced using urea as the feedstock. According to the reaction equation, six molecules of urea yield one molecule of melamine, six molecules of ammonia, and three molecules of carbon dioxide. Therefore, the system is filled with a mixed gas primarily composed of ammonia and carbon dioxide.

The production technology of melamine primarily consists of high-pressure and low-pressure methods. The high-pressure method involves high reaction pressure, and since the reaction takes place in the liquid phase, the density of reaction mass will be high, which will require more stringent construction material. After the reaction, the reaction mass containing melamine is scrubbed with ammonia water, whereby the melamine is absorbed into the liquid phase, followed by crystallization and purification to obtain solid melamine. This method has high energy consumption, requires large investment, and the associated technology, materials, and equipment are largely controlled by foreign companies. Domestic melamine production in China is mainly based on the low-pressure method. In addition to the relatively low reaction pressure, the most significant feature of the low-pressure method is that there is no liquid water involved in the entire process, the energy consumption is low, the material requirements are not high, all key apparatus and materials are domestically produced, and the investment is relatively low. To obtain solid melamine products after the reaction, the process gas containing gaseous melamine is directly contacted with a cold process gas for cooling. Melamine is condensed from gas to a solid, and then the solid melamine particles are separated from the process gas. Therefore, this method is also called a low-pressure gas-phase quenching method.

At present, within industry, the maximum capacity of a single low-pressure gas-phase quenching production line remains below the level of 60,000 tons per year. For many years, the 60,000-ton threshold has not been surpassed, and scale-up to 100,000 tons per year or beyond has been constrained by multiple factors, which are detailed below.

Firstly, an increase in production output requires a proportional increase in the mass flow rate of the feedstock, along with corresponding increases in the mass flow rates of the carrier gas and cooling gas. If parameters such as temperature and pressure remain unchanged, the density also remains unchanged, resulting in a proportional increase in the volumetric flow rate of the entire gas-phase circulation system. The reactor employed is a fluidized-bed reactor, which requires carrier gas to be introduced from the bottom. As the gas passes through the catalyst bed, it is expected to be distributed uniformly. The upward gas velocity within the fluidized-bed reactor must fall within a reasonable range, which determines the correlation between production capacity and reactor diameter. If output is increased and thus the volumetric flow rate of the carrier gas is increased, the reactor diameter must also be further enlarged. However, as the reactor diameter increases, achieving uniform gas distribution becomes increasingly difficult. Once maldistribution occurs, channeling may arise, and for a fluidized-bed reactor this leads to a rapid decline in reaction efficiency. Furthermore, for the reaction mass flowing through pipelines, under normal conditions, pressure drop (energy consumption) is proportional to the square of the flow velocity. To maintain relatively low energy consumption, if production capacity is scaled up while keeping flow velocity constant, the volumetric flow must remain constant, which means that an n-fold increase in capacity requires pipeline diameter to be enlarged by a factor of square root of n. For example, doubling the capacity requires the pipeline diameter to increase by a square root of 2 (1.414) times. Taking into account that an increase in pipe diameter also necessitates an increase in wall thickness, the investment in pipelines may rise by more than 1.5 times. Similar considerations apply to various other items of equipment. Overall, doubling the production capacity typically results in approximately a 1.5-fold increase in plant capital investment.

In addition, by maintaining a constant reaction temperature and volumetric flow rate, production output can also be increased by raising the reaction pressure, thereby increasing the mass flow rate. Since the volumetric flow remains unchanged, this approach does not require enlargement of equipment or pipeline dimensions. However, in practice, the situation is not so simple. This method has its limitations, and if the pressure is increased blindly, other issues will arise, as detailed below.

In the gas-phase melamine process, during the hot-gas cooling stage, the gas containing melamine products must be cooled so that high-boiling-point by-products precipitate from the gas phase into the solid phase, after which the by-products are separated from the gas stream using a filter. However, if the cooling temperature is too low, melamine itself will also precipitate and be filtered out, resulting in unacceptable product loss. Thus, the degree of cooling is limited. When the reaction pressure is increased, the total pressure of the hot process gas increases, and the partial pressure of melamine also rises proportionally. Under such conditions, a higher temperature is required to maintain melamine in the gaseous state, forcing the hot-gas cooling stage to operate at an elevated temperature. In turn, when the crystallizer is confronted with higher-temperature hot process gas, a greater amount of cold gas is required to counteract it in order to keep the crystallizer temperature within a reasonable range. The increase in cold-gas demand results in higher power consumption for the cold-gas blower, thereby raising operating costs. Therefore, detailed calculations are necessary to optimize the combination that can both increase production capacity and remain technically feasible, without incurring additional energy consumption. Achieving this balance is not something that a regular practitioner in the field can readily accomplish.

Gas-phase quenching takes place in the crystallizer, where the cold process gas comes into direct contact with the hot process gas containing gaseous melamine products. The melamine is cooled and converted from the gaseous state into the solid state, becoming entrained in the process gas to form a gas-solid mixture, which exits from the bottom of the crystallizer and flows through a pipeline into a separator. In the separator, gas-solid separation occurs: the solids accumulate at the bottom of the separator and are discharged via a feeding mechanism. The pipeline connecting the crystallizer and the separator continuously conveys a two-phase gas-solid stream, in which solids tend to accumulate and cause blockages. This constitutes a major bottleneck limiting the operating cycle of existing melamine plants. If the production capacity of the unit exceeds the current level of 50,000 tons per year, the solid throughput in the pipeline between the crystallizer and separator increases correspondingly, thereby aggravating the blockage problem. This significantly shortens the operating cycle and leads to frequent shutdowns and restarts. The downtime required for maintenance and clearing blockages results in lost production time, and after restart, a transition period is needed to adjust from off-specification to the desired high standard. Consequently, frequent shutdowns and restarts seriously disrupt stable production, increase costs, and reduce output.

In addition, in all current melamine production lines, melamine of all particle sizes is mixed into a single product without particle-size classification, which limits its applications. Products from the high-pressure process have relatively large particle sizes and high bulk density, making them suitable only for producing melamine-formaldehyde resin adhesives, but unsuitable for direct-use applications such as powder fire extinguishing agents and fire-retardant coatings. Conventional low-pressure process products have smaller average particle sizes and can be applied in various fields; however, their particle-size distribution is excessively broad. As a result, when used in the production of melamine-formaldehyde resin adhesives, the consistency of the time required to reach the end point of reaction is poor, and the storage stability of the adhesive is limited.

In summary, in view of the difficulties in scaling up the capacity of a single unit in gas-phase quenching melamine production, as well as the shortcomings of the existing technologies, there is an urgent need in the art to develop a process and apparatus for the large-scale production of specialty melamine by means of gas-phase quenching.

### SUMMARY OF THE INVENTION

In view of the foregoing, the present application provides a process for producing specialty melamine by gas-phase quenching, which can achieve an annual production capacity of 100,000 tons or more with relatively low energy consumption, thereby filling the technical gap that the annual production capacity of the existing single set of melamine production device cannot exceed 60,000 tons.

Correspondingly, the present application also provides an apparatus for producing specialty melamine by gas-phase quenching used in the aforementioned process.

In a first aspect, the present application provides a process for producing specialty melamine by gas-phase quenching, which comprises the following steps:
cooling hot process gas from an outlet of a melamine reactor in a hot gas cooler and filtering it in a hot gas filter successively, then admitting the cooled and filtered gas into a crystallizer where it directly contacts cold process gas, thereby producing solid A and mixed gas, discharging the solid A from the bottom of the crystallizer, and feeding the mixed gas into a separator to perform gas-solid separation and thereby obtaining solid B;
wherein the solid A is transported into a high-pressure collecting device A using high-pressure conveying gas A, and a temperature of the high-pressure conveying gas A is ensured to be 120°C or above during transportation; after performing gas-solid separation in the high-pressure collecting device A, the solid A is transported into a packaging device A using low-pressure conveying gas A, and a temperature of the solid A at the end of transportation pipe is ensured not to exceed 85°C; and a pressure of the high-pressure conveying gas A is in a range from 2 barG to 8 barG and is lower than a pressure inside the crystallizer, and a pressure of the low-pressure conveying gas A is in a range from 0.2 barG to 6.5 barG and is lower than a pressure of the high-pressure conveying gas A.

According to the production process of the present application, the solid A formed in the crystallizer by the deposition of melamine is discharged promptly, and only the fine particles that cannot settle to the bottom of the crystallizer will be carried to the separator by the process gas (i.e. the mixed gas referred to in the present application). On one hand, this configuration significantly reduces the burden of the pipeline between the crystallizer and the separator, thereby preventing blockage of the pipeline. On the other hand, due to the large particle size of the material collected to the bottom of the crystallizer and the small particle size of the material collected in the separator, automatic classification of the product material is achieved, thereby better meeting market demands, enhancing product competitiveness, and increasing economic benefits. Furthermore, according to the production process of the present application, the solid A discharged from the bottom of the crystallizer is transported in a two-stage (firstly transporting at high pressure and then transporting at low pressure). The pressure inside the high-pressure conveying pipe is relatively high, and the pressure difference between the crystallizer and the high-pressure conveying pipe is relatively small. When solid products are discharged from the bottom of the crystallizer, the amount of ammonia and carbon dioxide gas carried along with the solids is also very small. At the same time, the temperature of high-pressure conveying gas A is ensured to be 120°C or above during high-pressure transportation. This ensures that the ammonia and carbon dioxide gas escaping to the high-pressure conveying pipe can remain in gaseous state at a relatively high temperature, and will not deposit into the products, thereby avoiding any impact on the purity of the product. Subsequently, the ammonia and carbon dioxide gas are separated from the solid products in the high-pressure collector, therefore there is no ammonia and carbon dioxide gas when the solid reaches the low-pressure conveying pipe. At this point, there is no need to worry about deposition issues for ammonia and carbon dioxide during low-pressure and low-temperature conveying. Moreover, the final temperature of the product is relatively low, thereby preventing damage to packaging bags due to heat.

In an alternative embodiment, the process for producing specialty melamine by gas-phase quenching provided by the present application utilizes high-pressure conveying gas B to transport solid B into a high-pressure collecting device B, and a temperature of the high-pressure conveying gas B is ensured to be 120°C or above during transportation; after performing gas-solid separation in the high-pressure collecting device B, the solid B is transported into a packaging device B using low-pressure conveying gas B, and a temperature of solid B at the end of transportation pipe is ensured not to exceed 85°C; and a pressure of the high-pressure conveying gas B is in a range from 2 barG to 8 barG and is lower than a pressure inside the separator, and a pressure of the low-pressure conveying gas B is in a range from 0.2 barG to 6.5 barG and is lower than a pressure of the high-pressure conveying gas B.

The solid B separated by the separator is also transported in two stages (firstly transporting at high pressure and then transporting at low pressure). As mentioned above, it not only ensures product purity but also avoids damaging the packaging bags due to heat.

In an alternative embodiment, in the process for producing specialty melamine by gas-phase quenching provided by the present application, a mass ratio of the cold process gas to the hot process gas which enter the crystallizer is in a range from 2.6 to 3.2.

In an alternative embodiment, in the process for producing specialty melamine by gas-phase quenching provided in the present application, a pressure inside the crystallizer is in a range from 4 barG to 9.8 barG.

In an alternative embodiment, in the process for producing specialty melamine by gas-phase quenching provided in the present application, a discharge amount from the bottom of the crystallizer accounts for 5% to 50% of the total amount of solids produced.

In an alternative embodiment, in the process for producing specialty melamine by gas-phase quenching provided in the present application, a mass ratio of carrier gas to urea which enter the melamine reactor is in a range from 3.1 to 4.5.

In an alternative embodiment, in the process for producing specialty melamine by gas-phase quenching provided in the present application, the top of the melamine reactor has a temperature ranging from 380°C to 405°C, and the top of the melamine reactor has a pressure ranging from 5 barG to 10 barG.

The production process of the present application, by controlling the process parameters of the melamine reactor, hot gas cooler, hot gas filter, crystallizer, and separator, can significantly increase yield without increasing the size of the reactor, thereby avoiding engineering difficulties.

In a second aspect, the present application also provides an apparatus for producing specialty melamine by gas-phase quenching, which comprises:
a crystallizer discharging unit, comprising a crystallizer discharging device, a high-pressure collecting device A and a packaging device A, wherein an inlet of the crystallizer discharging device is connected to the bottom of the crystallizer, and an outlet of the crystallizer discharging device is connected to an inlet of the high-pressure collecting device A through a high-pressure conveying pipe A; a lower outlet of the high-pressure collecting device A is connected to the packaging device A through a low-pressure conveying pipe A; an outer wall of the high-pressure conveying pipe A is equipped with a heat preservation structure to ensure that a temperature inside the high-pressure conveying pipe A is 120°C or above, and a pressure inside the high-pressure conveying pipe A is in a range from 2 barG to 8 barG and is lower than a pressure inside the crystallizer; an outer wall of the low-pressure conveying pipe A is equipped with a cooling structure to ensure that a temperature inside the end of the low-pressure conveying pipe A does not exceed 85°C, and a pressure of the low-pressure conveying pipe A is in a range from 0.2 barG to 6.5 barG and is lower than the pressure inside the high-pressure conveying pipe A; and
a separator discharging unit, comprising a separator discharging device and a packaging device B, wherein an inlet of the separator discharging device is connected to the bottom of the separator, and an outlet of the separator discharging device is connected to the packaging device B, and the inlet of the separator is connected to an upper outlet of the crystallizer.

According to the production apparatus of the present application, the solid A, which is formed due to the deposition of melamine in a crystallizer, is promptly discharged, and only fine particles that cannot settle to the bottom of the crystallizer will be carried to the separator by the process gas (i.e. the mixed gas referred to in the present application). On one hand, this configuration significantly reduces the burden of the pipeline between the crystallizer and the separator, thereby preventing blockage of the pipeline. On the other hand, due to the large particle size of the material collected to the bottom of the crystallizer and the small particle size of the material collected in the separator, automatic classification of the product material is achieved, thereby better meeting market demands, enhancing product competitiveness, and increasing economic benefits. Furthermore, according to the production apparatus of the present application, the solid A discharged from the bottom of the crystallizer is transported in a two-stage (firstly transporting at high pressure and then transporting at low pressure). The pressure inside the high-pressure conveying pipe is relatively high, and the pressure difference between the crystallizer and the high-pressure conveying pipe is relatively small. When solid products are discharged from the bottom of the crystallizer, the amount of ammonia and carbon dioxide gas carried along with the solids is also very small. Additionally, the outer wall of the high-pressure conveying pipe is equipped with a heat preservation structure to ensure that the temperature of the high-pressure conveying gas A is 120°C or above during high-pressure conveying. This ensures that the ammonia and carbon dioxide gas escaping from the high-pressure conveying pipe can remain in gaseous state at a relatively high temperature, and will not condense into the products, thereby avoiding any impact on the purity of the product. Subsequently, the ammonia and carbon dioxide gas are separated from the solid products in the high-pressure collector, therefore there is no ammonia and carbon dioxide gas when the solid reaches the low-pressure conveying pipe (whose outer wall is equipped with a cooling structure). At this point, there is no need to worry about deposition issues for ammonia and carbon dioxide during low-pressure and low-temperature conveying. Moreover, the final temperature of the product is relatively low, thereby preventing damage to packaging bags due to heat.

In an alternative embodiment, in the apparatus for producing specialty melamine by gas-phase quenching provided by the present application, the crystallizer discharging device is a screw conveyor. Since the gas phase pressure inside the crystallizer is slightly higher than the pressure inside the high-pressure conveying pipe, the process gas (mainly ammonia and carbon dioxide) inside the crystallizer inevitably leaks along the screw direction towards the high-pressure conveying pipe. The advantage of using a screw conveyor is that inside the screw conveyor, the screw propels and compresses the product particles, reducing the gaps among particles and narrowing the passage for process gas flow, thereby reducing the amount of leaked process gas.

In an alternative embodiment, in the production apparatus for producing specialty melamine by gas-phase quenching provided by the present application, the outlet of the separator discharging device is connected to the inlet of the high-pressure collecting device B through the high-pressure conveying pipe B, and a lower outlet of the high-pressure collecting device B is connected to the packaging device B through a low-pressure conveying pipe B; and
an outer wall of the high-pressure conveying pipe B is equipped with a heat preservation structure to ensure that a temperature inside the high-pressure conveying pipe B is 120°C or above, and a pressure inside the high-pressure conveying pipe B is in a range from 2 barG to 8 barG and is lower than the pressure inside the separator; and an outer wall of the low-pressure conveying pipe B is equipped with a cooling structure to ensure that a temperature inside the end of the low-pressure conveying pipe B does not exceed 85°C, and a pressure inside the low-pressure conveying pipe B is in a range from 0.2 barG to 6.5 barG and is lower than the pressure inside the high-pressure conveying pipe B.

The solid B separated by the separator is also transported in two stages (firstly transporting at high pressure and then transporting at low pressure). As mentioned above, it not only ensures product purity but also avoids damaging the packaging bags due to heat.

In an alternative embodiment, the production apparatus for producing specialty melamine by gas-phase quenching provided by the present application further comprises: a carrier gas compressor, a carrier gas preheater, a melamine reactor, a hot gas cooler, a crystallizer, a separator, a urea scrubber and a cold gas blower which are sequentially connected;
an upper outlet of the separator is connected to the urea scrubber, to cool the gas to produce recyclable cold process gas; and
a cold gas outlet of the urea scrubber is connected to an inlet of the cold gas blower, and an outlet of the cold gas blower is connected to a cold process gas inlet of the crystallizer.

The production apparatus in the present application enables the recycling and utilization of cold process gas, thereby reducing production costs.

### BRIEF DESCRIPTION OF THE DRAWINGS

To more clearly illustrate the specific embodiments of the present application or the technical solutions in the prior art, the accompanying drawings required for the description of the specific embodiments or the prior art will be briefly introduced below. It is obvious that the accompanying drawings described below are some embodiments of the present application. For those skilled in the art, other drawings can be obtained based on these accompanying drawings without creative effort.
FIG. 1 is the production process flowchart of Example 1 of the present application.
FIG. 2 is a structural schematic diagram of the crystallizer discharging unit in Example 1 of the present application.
FIG. 3 is a structural schematic diagram of the separator discharging unit in Example 1 of the present application.

Among them, the reference signs are explained as follows:
1. Carrier gas compressor; 2. Carrier gas preheater; 3. Melamine reactor; 4. Hot gas cooler; 5. Hot gas filter; 6. Crystallizer; 7. Separator; 8. Urea scrubber; 9. Cold gas blower;
61. Crystallizer discharging device; 62. High-pressure conveying pipe A; 63. High-pressure collecting device A; 64. Low-pressure conveying pipe A; 65. Packaging device A;
71. Separator discharging device; 72. High-pressure conveying pipe B; 73. High-pressure collecting device B; 74. Low-pressure conveying pipe B; 75. Packaging device B.

### DETAILED DESCRIPTION

The following examples are provided for better further understanding of the present application, and are not limited to the described optimal embodiments. They do not constitute restrictions on the content and scope of protection of the present application. Any product that is identical or similar to the present application, whether derived from the insights of the present application or by combining the present application with features of other existing technologies, falls within the scope of protection of the present application.

If specific experimental steps or conditions are not specified in the examples, the operation or conditions can be conducted according to the conventional experimental steps described in the literature within the field. If the manufacturers of the reagents or instruments used are not specified, they are all conventional reagent and products that can be purchased commercially.

In the present application, the term "specialty melamine" refers to melamine products that can meet the specialized needs of different application scenarios.

To address the aforementioned issues present in the related technologies, according to the first aspect of the present application, a process for producing specialty melamine by gas-phase quenching is provided, which comprises the following steps:
cooling hot process gas from an outlet of a melamine reactor in a hot gas cooler and filtering it in a hot gas filter successively, then admitting the cooled and filtered gas into a crystallizer where it directly contacts cold process gas, thereby producing solid A and mixed gas, discharging the solid A from the bottom of the crystallizer, and feeding the mixed gas into a separator to perform gas-solid separation and thereby obtaining solid B;
wherein the solid A is transported into a high-pressure collecting device A using high-pressure conveying gas A, and a temperature of the high-pressure conveying gas A is ensured to be 120°C or above during transportation. As an example, the temperature of the high-pressure conveying gas A can be, for example, 120°C, 130°C, 140°C, 150°C, 160°C, 180°C, 200°C, or within a range consisting of any of the above values. After performing gas-solid separation in the high-pressure collecting device A, the solid A is transported into a packaging device A using low-pressure conveying gas A, and a temperature of the solid A at the end of transportation pipe is ensured not to exceed 85°C. As an example, the temperature of solid A at the end of transportation pipe can be, for example, 85°C, 75°C, 65°C, 55°C, 45°C, 35°C, 25°C, or within a range consisting of any of the above values. The pressure of the high-pressure conveying gas A is in a range from 2 barG to 8 barG and is lower than the pressure inside the crystallizer. As an example, the pressure of the high-pressure conveying gas A can be, for example, 2 barG, 3 barG, 4 barG, 5 barG, 6 barG, 7 barG, 8 barG, or within a range consisting of any of the above values. The pressure of the low-pressure conveying gas A is in a range from 0.2 barG to 6.5 barG and is lower than the pressure of the high-pressure conveying gas A. As an example, the pressure of the low-pressure conveying gas A can be, for example, 0.2 barG, 0.5 barG, 1 barG, 2 barG, 3 barG, 4 barG, 5 barG, 5.5 barG, 6 barG, 6.5 barG, or within a range consisting of any of the above values.

According to the production process of the present application, the solid A formed in the crystallizer by the deposition of melamine is discharged in a timely manner, and only the fine particles that cannot settle to the bottom of the crystallizer will be carried to the separator by the process gas (i.e. the mixed gas referred to in the present application). On one hand, this configuration significantly reduces the burden on the pipeline between the crystallizer and the separator, thereby preventing blockage of the pipeline. On the other hand, due to the large particle size of the material collected to the bottom of the crystallizer and the small particle size of the material collected in the separator, automatic classification of the product material is achieved, thereby better meeting market demands, enhancing product competitiveness, and increasing economic benefits. Furthermore, according to the production process of the present application, the solid A discharged from the bottom of the crystallizer is transported in a two-stage (firstly transporting at high pressure and then transporting at low pressure). The pressure inside the high-pressure conveying pipe is relatively high, and the pressure difference between the crystallizer and the high-pressure conveying pipe is relatively small. When solid products are discharged from the bottom of the crystallizer, the amount of ammonia and carbon dioxide gas carried along with the solids is also very small. At the same time, the temperature of the solid A is ensured to be 120°C or above during high-pressure transportation. This ensures that the ammonia and carbon dioxide gas escaping from the high-pressure conveying pipe can remain in gaseous state at a relatively high temperature, and will not condense into the products, thereby avoiding any impact on the purity of the product. Subsequently, the ammonia and carbon dioxide gas are separated from the solid products in the high-pressure collector, therefore there is no ammonia and carbon dioxide gas when the solid reaches the low-pressure conveying pipe. At this point, there is no need to worry about deposition issues for ammonia and carbon dioxide during low-pressure and low-temperature conveying. Moreover, the final temperature of the product is relatively low, thereby preventing damage to packaging bags due to heat.

It is understandable that the term "large-scale" mentioned in the present application refers to an annual production capacity of over 60,000 tons, such as 70,000 tons, 80,000 tons, 100,000 tons, 150,000 tons, 200,000 tons, etc., or within a range consisting of any of the above values.

It should be noted that traditional crystallizers completely disregard the issue of crystallizer discharge, with the gas outlet set at the center of the crystallizer bottom and discharging downward. Even if some solids within the crystallizer have already separated from the gas phase, they cannot be retained and will still flow out of the crystallizer with the gas. However, in the present application, by using technical means commonly known to those skilled in the art to modify and optimize the lower structure of the crystallizer, solid products can be collected and extracted at the bottom of the crystallizer. Therefore, the present application does not require any specific structural limitations on the crystallizer, as long as any crystallizer structure that can achieve the separation effect of the present application can be applied to the present application.

In an alternative embodiment, in the process for producing specialty melamine by gas-phase quenching provided in the present application, the solid B is transported into a high-pressure collecting device B using high-pressure conveying gas B, and a temperature of the high-pressure conveying gas B is ensured to be 120°C or above during transportation. As an example, the temperature of the high-pressure conveying gas B can be, for example, 120°C, 130°C, 140°C, 150°C, 160°C, 180°C, 200°C, etc., or within a range consisting of any of the above values. After performing gas-solid separation in the high-pressure collecting device B, the solid B is transported into a packaging device B using low-pressure conveying gas B, and a temperature of solid B at the end of transportation pipe is ensured not to exceed 85°C. As an example, the temperature of solid B at the end of the transportation pipe can be, for example, 85°C, 75°C, 65°C, 55°C, 45°C, 35°C, 25°C, etc., or within a range consisting of any of the above values. The pressure of the high-pressure conveying gas B is in a range from 2 barG to 8 barG and is lower than the pressure inside the separator. As an example, the pressure of the high-pressure conveying gas B can be, for example, 2 barG, 3 barG, 4 barG, 5 barG, 6 barG, 7 barG, 8 barG, etc., or within a range consisting of any of the above values. The pressure of the low-pressure conveying gas B is in a range from 0.2 barG to 6.5 barG and is lower than the pressure of the high-pressure conveying gas B. As an example, the pressure of the low-pressure conveying gas B can be, for example, 0.2 barG, 0.5 barG, 1 barG, 2 barG, 3 barG, 4 barG, 5 barG, 5.5 barG, 6 barG, 6.5 barG, etc., or within a range consisting of any of the above values.

The solid B separated by the separator is also transported in two stages (firstly transporting at high pressure and then transporting at low pressure). As mentioned above, it not only ensures product purity but also avoids damaging the packaging bags due to heat.

In an alternative embodiment, in the process for producing specialty melamine by gas-phase quenching provided by the present application, a mass ratio of the cold process gas to the hot process gas which enter the crystallizer is in a range from 2.6 to 3.2. As an example, the mass ratio of the cold process gas to the hot process gas which enter the crystallizer can be, for example, 2.6, 2.7, 2.8, 2.9, 3.0, 3.1, 3.2, etc., or within a range consisting of any of the above numerical values.

In an alternative embodiment, in the process for producing specialty melamine by gas-phase quenching provided by the present application, the pressure inside the crystallizer is in a range from 4 barG to 9.8 barG. As an example, the pressure inside the crystallizer can be, for example, 4 barG, 4.5 barG, 5 barG, 5.5 barG, 6 barG, 6.5 barG, 7 barG, 7.5 barG, 8 barG, 8.5 barG, 9 barG, 9.8 barG, etc., or within a range consisting of any of the above values.

In an alternative embodiment, in the process for producing specialty melamine by gas-phase quenching provided by the present application, a discharge amount from the bottom of the crystallizer accounts for 5% to 50% of the total amount of solids produced. As an example, the discharge amount from the bottom of the crystallizer accounts for 5%, 10%, 20%, 30%, 40%, 50%, etc. of the total amount of solids produced, or fall within a range consisting of any of the above values.

In an alternative embodiment, in the process for producing specialty melamine by gas-phase quenching provided by the present application, a mass ratio of carrier gas to urea which enter the melamine reactor is in a range from 3.1 to 4.5. As an example, the mass ratio of carrier gas to urea which enter the melamine reactor can be, for example, 3.1, 3.3, 3.5, 3.7, 3.9, 4.1, 4.3, 4.5, etc., or fall within a range consisting of any of the above numerical values.

In an alternative embodiment, in the process for producing specialty melamine by gas-phase quenching provided by the present application, the top of the melamine reactor has a temperature ranging from 380°C to 405°C, and the top of the melamine reactor has a pressure ranging from 5 barG to 10 barG. As an example, the top of the melamine reactor has a temperature which can be 380°C, 385°C, 390°C, 395°C, 400°C, 405°C, etc., or within a range consisting of any of the above values; the top of the melamine reactor has a pressure which can be 5 barG, 6 barG, 7 barG, 8 barG, 9 barG, 10 barG, etc., or within a range consisting of any of the above values.

The production process of the present application, by controlling the above process parameters of the melamine reactor, hot gas cooler, hot gas filter, crystallizer, and separator, can significantly increase production capacity without increasing the size of the reactor, thereby avoiding engineering difficulties.

According to the second aspect of the present application, there is also provided a production apparatus for producing specialty melamine by gas-phase quenching, which comprises:
a crystallizer discharging unit, comprising a crystallizer discharging device, a high-pressure collecting device A and a packaging device A, wherein an inlet of the crystallizer discharging device is connected to the bottom of the crystallizer, and an outlet of the crystallizer discharging device is connected to an inlet of the high-pressure collecting device A through a high-pressure conveying pipe A; a lower outlet of the high-pressure collecting device A is connected to the packaging device A through a low-pressure conveying pipe A; an outer wall of the high-pressure conveying pipe A is equipped with a heat preservation structure; an outer wall of the low-pressure conveying pipe A is equipped with a cooling structure; and
a separator discharging unit, comprising a separator discharging device and a packaging device B, wherein an inlet of the separator discharging device is connected to the bottom of the separator, and an outlet of the separator discharging device is connected to the packaging device B, and the inlet of the separator is connected to an upper outlet of the crystallizer.

Further, in an alternative embodiment, the production apparatus for producing specialty melamine by gas-phase quenching comprises:
a crystallizer discharging unit, comprising a crystallizer discharging device, a high-pressure collecting device A and a packaging device A, wherein an inlet of the crystallizer discharging device is connected to the bottom of the crystallizer, and an outlet of the crystallizer discharging device is connected to an inlet of the high-pressure collecting device A through a high-pressure conveying pipe A; a lower outlet of the high-pressure collecting device A is connected to the packaging device A through a low-pressure conveying pipe A; an outer wall of the high-pressure conveying pipe A is equipped with a heat preservation structure to ensure that a temperature inside the high-pressure conveying pipe A is 120°C or above; as an example, the temperature of the high-pressure conveying pipe A may be, for example, 120°C, 130°C, 140°C, 150°C, 160°C, 180°C, 200°C, etc., or within a range consisting of any of the above values, and a pressure inside the high-pressure conveying pipe A is in a range from 2 barG to 8 barG and is lower than a pressure inside the crystallizer; as an example, the pressure inside the high-pressure conveying pipe A may be, for example, 2 barG, 3 barG, 4 barG, 5 barG, 6 barG, 7 barG, 8 barG, etc., or within a range consisting of any of the above values; an outer wall of the low-pressure conveying pipe A is equipped with a cooling structure to ensure that a temperature inside the end of the low-pressure conveying pipe A does not exceed 85°C; as an example, the temperature inside the end of the low-pressure conveying pipe A may be, for example, 85°C, 75°C, 65°C, 55°C, 45°C, 35°C, 25°C, etc., or within a range consisting of any of the above values; and a pressure of the low-pressure conveying pipe A is in a range from 0.2 barG to 6.5 barG and is lower than a pressure inside the high-pressure conveying pipe A; as an example, the pressure inside the low-pressure conveying pipe A may be, for example, 0.2 barG, 0.5 barG, 1 barG, 2 barG, 3 barG, 4 barG, 5 barG, 5.5 barG, 6 barG, 6.5 barG, etc., or within a range consisting of any of the above values; and
a separator discharging unit, comprising a separator discharging device and a packaging device B, wherein an inlet of the separator discharging device is connected to the bottom of the separator, and an outlet of the separator discharging device is connected to the packaging device B, and the inlet of the separator is connected to an upper outlet of the crystallizer.

According to the production apparatus of the present application, the solid A, which is formed due to the deposition of melamine in a crystallizer, is promptly discharged, and only fine particles that cannot settle to the bottom of the crystallizer will be carried to the separator by the process gas (i.e. the mixed gas referred to in the present application). On one hand, this configuration significantly reduces the burden on the pipeline between the crystallizer and the separator, thereby preventing blockage of the pipeline. On the other hand, due to the large particle size of the material collected to the bottom of the crystallizer and the small particle size of the material collected in the separator, automatic classification of the product material is achieved, thereby better meeting market demands, enhancing product competitiveness, and increasing economic benefits. Furthermore, according to the production apparatus of the present application, the solid A discharged from the bottom of the crystallizer is transported in a two-stage (firstly transporting at high pressure and then transporting at low pressure). The pressure inside the high-pressure conveying pipe is relatively high, and the pressure difference between the crystallizer and the high-pressure conveying pipe is relatively small. When solid products are discharged from the bottom of the crystallizer, the amount of ammonia and carbon dioxide gas carried along with the solids is also very small. Additionally, the outer wall of the high-pressure conveying pipe is equipped with a heat preservation structure to ensure that the temperature of the solid A is 120°C or above during high-pressure conveying. This ensures that the ammonia and carbon dioxide gas escaping from the high-pressure conveying pipe can remain in gaseous state at a relatively high temperature, and will not condense into the products, thereby avoiding any impact on the purity of the product. Subsequently, the ammonia and carbon dioxide gas are separated from the solid products in the high-pressure collector, therefore there is no ammonia and carbon dioxide gas when the solid reaches the low-pressure conveying pipe (whose outer wall is equipped with a cooling structure). At this point, there is no need to worry about deposition issues for ammonia and carbon dioxide during low-pressure and low-temperature conveying. Moreover, the final temperature of the product is relatively low, thereby preventing damage to packaging bags due to heat.

In an alternative embodiment, in the apparatus for producing specialty melamine by gas-phase quenching provided by the present application, the crystallizer discharging device is a screw conveyor. Since the gas phase pressure inside the crystallizer is slightly higher than the pressure inside the high-pressure conveying pipe, the process gas (mainly ammonia and carbon dioxide) inside the crystallizer inevitably leaks along the screw direction towards the high-pressure conveying pipe. The advantage of using a screw conveyor is that inside the screw conveyor, the screw propels and compresses the product particles, reducing the gaps among particles and narrowing the passage for process gas flow, thereby reducing the amount of leaked process gas.

In an alternative embodiment, in the production apparatus for producing specialty melamine by gas-phase quenching provided by the present application, the outlet of the separator discharging device is connected to the inlet of the high-pressure collecting device B through the high-pressure conveying pipe B, and a lower outlet of the high-pressure collecting device B is connected to the packaging device B through a low-pressure conveying pipe B; and
an outer wall of the high-pressure conveying pipe B is equipped with a heat preservation structure to ensure that a temperature inside the high-pressure conveying pipe B is 120°C or above; as an example, the temperature of the high-pressure conveying pipe B can be, for example, 120°C, 130°C, 140°C, 150°C, 160°C, 180°C, 200°C, etc., or within a range consisting of any of the above values; and a pressure inside the high-pressure conveying pipe B is in a range from 2 barG to 8 barG and is lower than the pressure inside the separator; as an example, the pressure of the high-pressure conveying pipe B can be, for example, 2 barG, 3 barG, 4 barG, 5 barG, 6 barG, 7 barG, 8 barG, etc., or within a range consisting of any of the above values; and the outer wall of the low-pressure conveying pipe B is equipped with a cooling structure to prevent the temperature inside the end of the low-pressure conveying pipe B does not exceed 85°C; as an example, the temperature inside the end of the low-pressure conveying pipe B can be, for example, 85°C, 75°C, 65°C, 55°C, 45°C, 35°C, 25°C, etc., or within a range consisting of any of the above values; and the pressure inside the low-pressure conveying pipe B is in a range from 0.2 barG to 6.5 barG and is lower than the pressure inside the high-pressure conveying pipe B; as an example, the pressure inside the low-pressure conveying pipe B can be, for example, 0.2 barG, 0.5 barG, 1 barG, 2 barG, 3 barG, 4 barG, 5 barG, 5.5 barG, 6 barG, 6.5 barG, etc., or within a range consisting of any of the above values.

The solid B separated by the separator is also transported in two stages (firstly transporting at high pressure and then transporting at low pressure). As mentioned above, it not only ensures product purity but also avoids damaging the packaging bags due to heat.

In an alternative embodiment, the production apparatus for producing specialty melamine by gas-phase quenching provided by the present application further comprises: a carrier gas compressor, a carrier gas preheater, a melamine reactor, a hot gas cooler, a crystallizer, a separator, a urea scrubber and a cold gas blower which are sequentially connected;
an upper outlet of the separator is connected to the urea scrubber, to cool the gas to produce recyclable cold process gas; and
a cold gas outlet of the urea scrubber is connected to an inlet of the cold gas blower, and an outlet of the cold gas blower is connected to a cold process gas inlet of the crystallizer.

The production apparatus in the present application enables the recycling and utilization of cold process gas, thereby reducing production costs.

The present application is further described in detail below with reference to specific examples, which should not be construed as limiting the scope of protection claimed by the present application.

### Example 1

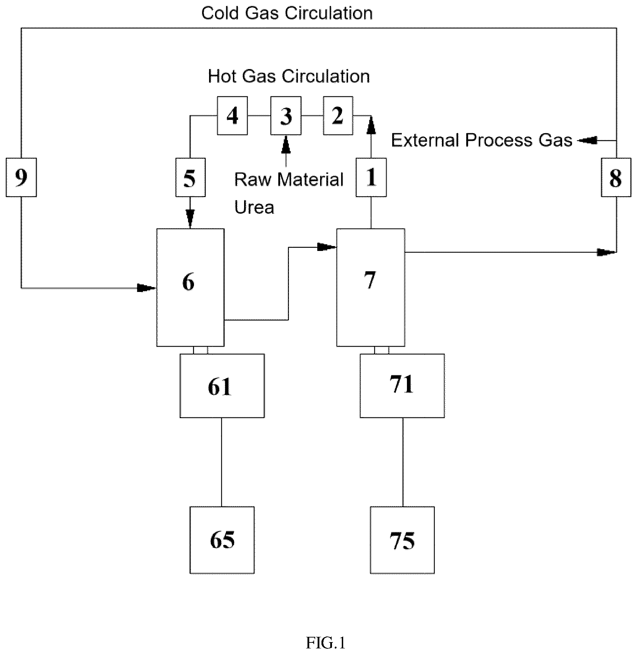

This example provides a production process for large-scale production (100,000 tons/year) of specialty melamine using gas-phase quenching, as shown in FIG. 1, which comprises the following steps:
117 t/h of hot process gas was pressurized to 7 bar(G) by carrier gas compressor 1 and heated to 405°C by carrier gas preheater 2 before feeding the melamine reactor 3 as fluidized carrier gas. 37.5 t/h of raw material urea was fed into the melamine reactor 3 (the mass ratio of carrier gas to urea was 3.12). 3 tons of urea produced 1 ton of melamine, and all products other than melamine were represented by ammonia and carbon dioxide. In this example and the comparative examples, the molar ratio of ammonia to carbon dioxide in various process gases within the entire system was considered to be a theoretical value of 2:1. The efficiency of carrier gas compressor 1 was calculated at 80%, and the efficiency of cold gas blower 9 was calculated at 70%. The outlet of melamine reactor 3 obtained 154.5 t/h of hot process gas, with a temperature of 405°C and a pressure of 6 bar(G).

The hot process gas at the outlet of melamine reactor 3 contained 12.5 t/h of melamine, equivalent to a mole fraction of 1.78%. The total pressure was 6 bar(G), the partial pressure was calculated to be 0.125 bar(A). At this pressure, the theoretical temperature for melamine condensation (deposition) was calculated to be 337°C, leaving a margin of 20°C. The operating temperature of hot gas cooler 4 was set at 357°C. The hot process gas at the outlet of hot gas cooler 4 further passed through hot gas filter 5, reducing the pressure to 5.7 bar(G), before entering crystallizer 6. Simultaneously, cold process gas was fed into crystallizer 6 by cold gas blower 9 to ensure a mass ratio of 2.65 of the cold process gas to the hot process gas. After mixing in crystallizer 6, the temperature of the hot and cold process gases became 215°C, and the pressure dropped to 5.67 bar(G), resulting in the crystallization of melamine into a solid.

The power of the cold gas blower 9 was 923 kW, and the power of the carrier gas compressor 1 was 1,364 kW. According to the formula: energy consumption per unit product = (power of cold gas blower + power of carrier gas compressor) / melamine production, the energy consumption per unit product in this example was calculated to be 183 kWh/t of melamine.

6.25 t/h of melamine solids were discharged from the bottom of crystallizer 6. In the pipeline from crystallizer 6 to separator 7, there were 6.25 t/h of melamine solids and 552 t/h of process gas, with a gas-solid ratio of 88. The higher the gas-solid ratio, the less likely the solids were to accumulate and block in the pipeline. This process gas easily carried away the solids, making it less likely for them to deposit in the pipeline from crystallizer 6 to separator 7.

The pressure of the process gas at the outlet of separator 7 was reduced to 5.5 bar(G), and the pressure of the process gas after passing through urea scrubber 8 was reduced to 5.42 bar(G). In the present application, the top pressure of melamine reactor 3 was taken as a representative of the system pressure. All the above pressure values constituted a set of correlated parameters. In subsequent examples and comparative examples, when the top pressure of the melamine reactor was changed, all other pressure values were changed accordingly.

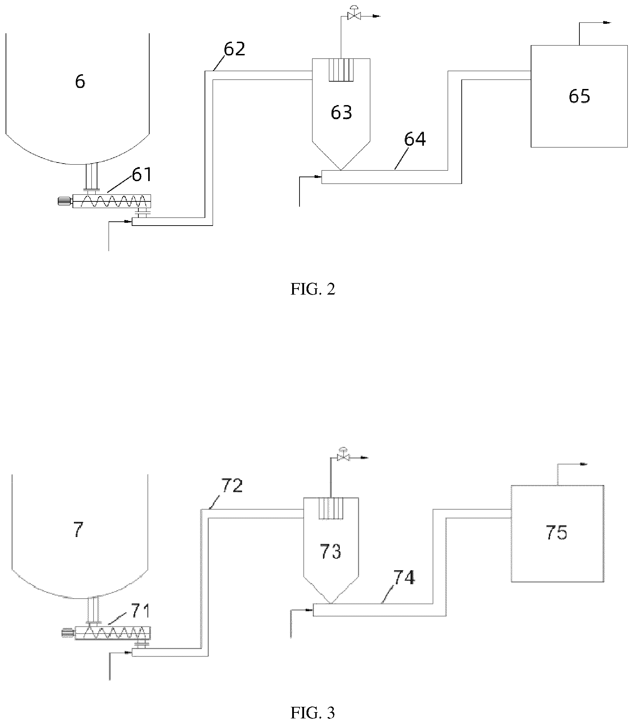

Referring to FIG. 2, 6.25 t/h of melamine solids were discharged from the crystallizer discharging device 61 at the bottom of the crystallizer 6, transported using 4 t/h of air. The pressure inside the high-pressure conveying pipe A 62 was maintained at 2.5 bar(G), and insulation cotton was applied externally to the high-pressure conveying pipe A 62 to reduce heat dissipation. The temperature at the end of the high-pressure conveying pipe A 62 was maintained at 140°C or above. After reaching the high-pressure collector A 63, 4 t/h of air was separated from the melamine solids, and the solids were collected at the bottom of the high-pressure collector A 63. Then, another 4 t/h of air was used for transportation, and the pressure inside the low-pressure conveying pipe A 64 was controlled at 0.2 bar(G). Circulating water jacket was applied externally to the low-pressure conveying pipe A 64 for cooling, and the temperature at the end of the low-pressure conveying pipe A 64 was reduced to 40°C, before reaching the packaging device A 65.

As shown in FIG. 3, the remaining 6.25 t/h of melamine solids were discharged from the separator discharging device 71 at the bottom of separator 7. They were conveyed using 3.5 t/h of air, with the pressure maintained at 2.5 bar(G) inside the high-pressure conveying pipe B 72. Insulation cotton was applied externally to the high-pressure conveying pipe B 72 to reduce heat dissipation, and the temperature at the end of the high-pressure conveying pipe B 72 was maintained at 140°C or above. Upon reaching the high-pressure collector B 73, 3.5 t/h of air was separated from the melamine solids, and the solids were collected at the bottom of the high-pressure collector B 73 and conveyed again using a stream of 3.5 t/h of air. The pressure inside the low-pressure conveying pipe B 74 was controlled at 0.2 bar(G). Circulating water jacket was applied externally to the low-pressure conveying pipe B 74 for cooling, the temperature at the end of the pipe was reduced to 40°C, before reaching the packaging device B 75.

The packaging device B 75 produced fine-grained melamine products with a D50 of 15 microns to 25 microns and a bulk density of 0.4 g/cm³ to 0.6 g/cm³. The products were specifically used for the production of fireproof coatings, powder fire extinguishing agents, or melamine cyanurate salts, which require the use of fine particles for special applications.

The packaging device A 65 produced a coarse-particle melamine product with a D50 of 50 microns to 60 microns and a bulk density greater than 0.6g/cm³. This product was specifically used for applications requiring coarse particles, such as the production of melamine-formaldehyde resin glue. Compared to melamine products produced by conventional gas-phase quenching, this product contained fewer fine particles and contained coarse particles with narrower particle size distribution. This results in good consistency in the reaction endpoint when used in glue production, leading to stable glue performance.

Because the pressure difference among the crystallizer, separator, and their respective high-pressure conveying pipes were not big, the gas leakage rate at the bottom of both the crystallizer and separator was calculated to be 0.02%, with a total gas leakage volume of 0.11 t/h. Since the temperatures at the ends of high-pressure conveying pipe A and high-pressure conveying pipe B were relatively high (140°C or above), ammonia and carbon dioxide did not crystallize into ammonium carbamate but left the solid product in gaseous form from high-pressure collector A and high-pressure collector B. Then, during low-pressure conveying, the temperature drop did not cause ammonium carbamate to crystallize, and the purity of the final product remained unaffected. The product purity can reach 99.5%, which meets the requirements for superior quality products.

### Example 2

143 t/h of hot process gas was pressurized to 9.4 bar(G) by the carrier gas compressor 1 and heated to 380°C by the carrier gas preheater 2 before entering the melamine reactor 3 as fluidizing carrier gas. 37.5 t/h of raw material urea was fed into the melamine reactor 3 (the mass ratio of carrier gas to urea was 3.81). The outlet of the melamine reactor 3 produced 180.5 t/h of hot process gas, with a temperature of 380°C and a pressure of 8 bar(G).

The hot process gas at the outlet of melamine reactor 3 contained 12.5 t/h of melamine, equivalent to a mole fraction of 1.51%. The total pressure was 8 bar(G), the partial pressure was calculated to be 0.136 bar(A). At this pressure, the theoretical temperature for melamine condensation (deposition) was calculated to be 339°C, leaving a margin of 20°C. The operating temperature of hot gas cooler 4 was set at 359°C. The hot process gas at the outlet of hot gas cooler 4 was further passed through hot gas filter 5, reducing the pressure to 7.7 bar(G), before entering crystallizer 6. Simultaneously, cold process gas was fed into crystallizer 6 by cold gas blower 9 to ensure a mass ratio of 3.05 of the cold process gas to the hot process gas. After mixing in crystallizer 6, the temperature of the hot and cold process gases became 208°C, and the pressure dropped to 7.64 bar(G), resulting in the crystallization of melamine into a solid.

The power of the cold gas blower 9 was 1,225 kw, and the power of the carrier gas compressor 1 was 1,653 kw. According to the formula: energy consumption per unit product = (power of cold gas blower + power of carrier gas compressor) / melamine production, the energy consumption per unit product in this example was calculated to be 230 kwh/t of melamine.

4 t/h of melamine solids were discharged from the bottom of crystallizer 6. In the pipeline from crystallizer 6 to separator 7, there were 8.5 t/h of melamine solids and 718 t/h of process gas, with a high gas-solid ratio of 84. Due to this high ratio, the process gas tends to carry away the solids, making it difficult for them to deposit in the pipeline from crystallizer 6 to separator 7.

The pressure of process gas at the outlet of separator 7 was reduced to 7.44 bar(G), and the pressure of process gas after passing through urea scrubber 8 was reduced to 7.34 bar(G).

4 t/h of melamine solids were discharged from the crystallizer discharging device 61 at the bottom of crystallizer 6, transported using 2 t/h of air. The pressure inside the high-pressure conveying pipe A 62 was maintained at 4 bar(G), and insulation cotton was applied externally to reduce heat dissipation. The temperature at the end of high-pressure conveying pipe A 62 was maintained at 150°C or above. After reaching the high-pressure collector A 63, 2 t/h of air was separated from the melamine solids, and the solids were collected at the bottom of high-pressure collector A 63. Then, 2 t/h of air was used for transportation, and the pressure inside the low-pressure conveying pipe A 64 was controlled at 1.5 bar(G). Circulating water jacket was applied externally to the low-pressure conveying pipe A 64 for cooling, and the temperature at the end of low-pressure conveying pipe A 64 was reduced to 42°C, before reaching the packaging device A 65.

The remaining 8.5 t/h of melamine solids were discharged from the separator discharging device 71 at the bottom of separator 7. They were conveyed using 4 t/h of air, with the pressure maintained at 4 bar(G) inside the high-pressure conveying pipe B 72. Insulation cotton was applied externally to the high-pressure conveying pipe B 72 to reduce heat dissipation, and the temperature at the end of the high-pressure conveying pipe B 72 was maintained at 150°C or above. Upon reaching the high-pressure collector B 73, 4 t/h of air was separated from the melamine solids, and the solids were collected at the bottom of the high-pressure collector B 73 and then transported using 4 t/h of air. The pressure inside the low-pressure conveying pipe B 74 was controlled at 1.5 bar(G). Circulating water jacket was applied externally to the low-pressure conveying pipe B 74 for cooling, the temperature at the end of the low-pressure conveying pipe B 74 was reduced to 42°C, before reaching the packaging device B 75.

The packaging device B 75 produced fine-grained melamine products with a D50 of 15 microns to 25 microns and a bulk density of 0.4 g/cm³ to 0.6 g/cm³. The products were specifically used for the production of fireproof coatings, powder fire extinguishing agents, or melamine cyanurate salts, etc., which require the use of fine particles for special applications.

The packaging device A 65 produced a coarse-particle melamine product with a D50 of 50 to 60 microns and a bulk density greater than 0.6g/cm³. This product was specifically used in applications requiring coarse particles, such as the production of melamine-formaldehyde resin glue. Compared to melamine products produced by conventional gas-phase quenching, this product contained fewer fine particles and contained coarse particles with narrower particle size distribution. This results in good consistency in the reaction endpoint when used in glue production, leading to stable glue performance.

The temperatures inside high-pressure conveying pipe A and high-pressure conveying pipe B were relatively high, but similar to Example 1, this did not affect the purity of the product. The purity of the product can reach 99.5%, which meets the requirements for superior quality products.

### Example 3

168 t/h of hot process gas was pressurized to 11.75 bar(G) by carrier gas compressor 1 and heated to 395°C by carrier gas preheater 2 before entering the melamine reactor 3 as fluidizing carrier gas. 37.5 t/h of raw material urea was fed into the melamine reactor 3 (the mass ratio of carrier gas to urea was 4.48). The outlet of melamine reactor 3 produced 205.5 t/h of hot process gas, with a temperature of 395°C and a pressure of 10 bar(G).

The hot process gas at the outlet of melamine reactor 3 contained 12.5 t/h of melamine, equivalent to a mole fraction of 1.32%. The total pressure was 10 bar(G), the partial pressure was calculated to be 0.145 bar(A). At this pressure, the theoretical temperature for melamine condensation (deposition) was calculated to be 340°C, leaving a margin of 20°C. The operating temperature of hot gas cooler 4 was set at 360°C. The hot process gas at the outlet of hot gas cooler 4 further passed through hot gas filter 5 to reduce the pressure to 9.7 bar(G) before entering crystallizer 6. Meanwhile, cold process gas was sent into crystallizer 6 by cold gas blower 9 to ensure a mass ratio of 3.19 of the cold process gas to the hot process gas. After mixing in crystallizer 6, the temperature of the hot and cold process gases became 206°C, and the pressure dropped to 9.625 bar(G), resulting in the crystallization of melamine into a solid.

The power of the cold gas blower 9 was 1,467 kw, and the power of the carrier gas compressor 1 was 1,915 kw. According to the formula: energy consumption per unit product = (power of cold gas blower + power of carrier gas compressor) / melamine production, the energy consumption per unit product in this example was calculated to be 271 kwh/t melamine.

1.1 t/h of melamine solids were separated from the bottom of crystallizer 6. In the pipeline from crystallizer 6 to separator 7, there were 11.4 t/h of melamine solids and 848 t/h of process gas, with a gas-solid ratio of 74. The high gas-solid ratio made it easier for the process gas to carry away the solids, which were less likely to deposit in the pipeline from crystallizer 6 to separator 7.

The pressure of the process gas at the outlet of separator 7 was reduced to 9.365 bar(G), and the pressure of the process gas after passing through urea scrubber 8 was reduced to 9.255 bar(G).

1.1 t/h of melamine solids were discharged from the crystallizer discharging device 61 at the bottom of crystallizer 6, transported using 2 t/h of air. The pressure inside the high-pressure conveying pipe A 62 was maintained at 7 bar(G), and insulation cotton was applied externally to the high-pressure conveying pipe A 62 to reduce heat dissipation. The temperature at the end of the high-pressure conveying pipe A 62 was maintained at 130°C or above. After reaching the high-pressure collector A 63, 2 t/h of air was separated from the melamine solids, and the solids were collected at the bottom of the high-pressure collector A 63. Then, 3 t/h of air was used for transportation, and the pressure inside the low-pressure conveying pipe A 64 was controlled at 6 bar(G). Circulating water jacket was applied externally to the low-pressure conveying pipe A 64 for cooling, and the temperature at the end of the low-pressure conveying pipe A 64 was reduced to 50°C, before reaching the packaging device A 65.

The remaining 11.4 t/h of melamine solids were discharged from the separator discharging device 71 at the bottom of separator 7. They were conveyed using 7 t/h of air, with the pressure maintained at 7 bar(G) inside the high-pressure conveying pipe B 72. Insulation cotton was applied externally to the high-pressure conveying pipe B 72 to reduce heat dissipation, and the temperature at the end of the high-pressure conveying pipe B 72 was maintained at 130°C or above. Upon reaching the high-pressure collector B 73, 7 t/h of air was separated from the melamine solids, and the solids were collected at the bottom of the high-pressure collector B 73. Then, 7 t/h of air was used for transportation, with the pressure inside the low-pressure conveying pipe B 74 controlled at 6 bar(G). Circulating water jacket was applied externally to the low-pressure conveying pipe B 74 for cooling, and the temperature at the end of the low-pressure conveying pipe B 74 was reduced to 50°C, before reaching the packaging device B 75.

The packaging device B 75 produced fine-grained melamine products with a D50 of 15 microns to 25 microns and a bulk density of 0.4 g/cm³ to 0.6 g/cm³. The products were specifically used for the production of fireproof coatings, powder fire extinguishing agents, or melamine cyanurate salts, which require the use of fine particles for special applications.

The packaging device A 65 produced a coarse-particle melamine product with a D50 of 50 microns to 60 microns and a bulk density greater than 0.6g/cm³. This product was specifically used for applications requiring coarse particles, such as the production of melamine-formaldehyde resin glue. Compared to melamine products produced by conventional gas-phase quenching, this product contained fewer fine particles and are coarse particles with narrower particle size distribution. This results in good consistency in the reaction endpoint when used in glue production, leading to stable glue performance.

The temperatures inside high-pressure conveying pipe A and high-pressure conveying pipe B were relatively high, but similar to Example 1, this did not affect the purity of the product. The purity of the product can reach 99.5%, which meets the requirements of superior quality products.

### Example 4

A production apparatus for large-scale production of specialty melamine by gas-phase quenching, comprises a carrier gas compressor, a carrier gas preheater, a melamine reactor, a hot gas cooler, a crystallizer, a separator, a urea scrubber, and a cold gas blower, which were sequentially connected and arranged, and further comprises a crystallizer discharging unit and a separator discharging unit; wherein,
an upper outlet of the separator was connected to the urea scrubber, to cool the gas to produce recyclable cold process gas; and
a cold gas outlet of the urea scrubber is connected to an inlet of the cold gas blower, and the outlet of the cold gas blower is connected to a cold process gas inlet of the crystallizer;
the crystallizer discharging unit comprises a crystallizer discharging device 61, a high-pressure collecting device A 63, and a packaging device A 65, the inlet of the crystallizer discharging device 61 is connected to the bottom of the crystallizer 6, and the outlet of the crystallizer discharging device 61 is connected to the inlet of the high-pressure collecting device A 63 through a high-pressure conveying pipe A 62, the lower outlet of the high-pressure collecting device A 63 is connected to the packaging device A 65 through a low-pressure conveying pipe A 64; the outer wall of the high-pressure conveying pipe A is equipped with a heat preservation structure to ensure that the temperature in the high-pressure conveying pipe A is 120°C or above, and the pressure inside the high-pressure conveying pipe A is in a range from 2 barG and 8 barG and is lower than the pressure inside the crystallizer; the outer wall of the low-pressure conveying pipe A is equipped with a cooling structure to ensure that the temperature at the end of the low-pressure conveying pipe A did not exceed 85°C, and the pressure inside the low-pressure conveying pipe A is in a range from 0.2 barG and 6.5 barG and is lower than the pressure inside the high-pressure conveying pipe A. In this example, the crystallizer discharging device is a screw conveyor.

The separator discharging unit comprises a separator discharging device 71, a high-pressure collecting device B 73, and a packaging device B 75, the inlet of the separator discharging device 71 is connected to the bottom of the separator 7, and the inlet of the separator 7 is connected to the upper outlet of the crystallizer 6, the outlet of the separator discharging device 71 is connected to the inlet of the high-pressure collecting device B 73 through a high-pressure conveying pipe B 72, the lower outlet of the high-pressure collecting device B 73 is connected to the packaging device B 75 through a low-pressure conveying pipe B 74, the outer wall of the high-pressure conveying pipe B is equipped with a heat preservation structure to ensure that the temperature in the high-pressure conveying pipe B is 120°C or above, the pressure inside the high-pressure conveying pipe B is in a range from 2 barG to 8 barG and lower than the pressure inside the separator; the outer wall of the low-pressure conveying pipe B is equipped with a cooling structure to ensure that the temperature at the end of the low-pressure conveying pipe B did not exceed 85°C, the pressure inside the low-pressure conveying pipe B is in a range from 0.2 barG to 6.5 barG and lower than the pressure inside the high-pressure conveying pipe B.

### Example 5

A production apparatus for large-scale production of specialty melamine by gas-phase quenching, comprises a carrier gas compressor, a carrier gas preheater, a melamine reactor, a hot gas cooler, a crystallizer, a separator, a urea scrubber, and a cold gas blower which are sequentially connected and arranged, and further comprises a crystallizer discharging unit and a separator discharging unit; wherein,
an upper outlet of the separator is connected to the urea scrubber, to cool the gas to produce recyclable cold process gas; and
a cold gas outlet of the urea scrubber is connected to an inlet of the cold gas blower, and the outlet of the cold gas blower is connected to a cold process gas inlet of the crystallizer;
the crystallizer discharging unit comprises a crystallizer discharging device 61, a high-pressure collecting device A 63, and a packaging device A 65, the inlet of the crystallizer discharging device 61 is connected to the bottom of the crystallizer 6, and the outlet of the crystallizer discharging device 61 is connected to the inlet of the high-pressure collecting device A 63 through a high-pressure conveying pipe A 62, the lower outlet of the high-pressure collecting device A 63 is connected to the packaging device A 65 through a low-pressure conveying pipe A 64; the outer wall of the high-pressure conveying pipe A 62 is equipped with a heat preservation structure, and the outer wall of the low-pressure conveying pipe A 64 is equipped with a cooling structure.

The separator discharging unit comprises a separator discharging device 71, a high-pressure collecting device B 73, and a packaging device B 75, the inlet of the separator discharging device 71 is connected to the bottom of the separator 7, and the inlet of the separator 7 is connected to the upper outlet of the crystallizer 6, the outlet of the separator discharging device 71 is connected to the inlet of the high-pressure collecting device B 73 through a high-pressure conveying pipe B 72, the lower outlet of the high-pressure collecting device B 73 is connected to the packaging device B 75 through a low-pressure conveying pipe B 74; and the outer wall of the high-pressure conveying pipe B 72 is equipped with a heat preservation structure, and the outer wall of the low-pressure conveying pipe B 74 is equipped with a cooling structure.

### Comparative Example 1: annual production capacity of 50,000 tons (6.25 t/h)

The parameters of this comparative example were relatively close to those of a conventional 50,000-ton plant today. It should be noted that this comparative example was only a hypothetical 50,000-ton process flow and parameters, and did not represent the existence of corresponding process technology in reality.

85 t/h of hot process gas was pressurized to 4.4 bar(G) by carrier gas compressor 1, and heated to 401°C by carrier gas preheater 2 before entering melamine reactor 3 as fluidizing carrier gas. 18.75 t/h of raw material urea was fed into melamine reactor 3 (the mass ratio of carrier gas to urea was 4.53). The outlet of melamine reactor 3 obtained 103.75 t/h of hot process gas with a temperature of 401°C and pressure of 3.5 bar(G).

The hot process gas at the outlet of melamine reactor 3 contained 6.25 t/h of melamine, equivalent to a mole fraction of 1.19%. The total pressure was 3.5 bar(G), the partial pressure was calculated to be 0.054 bar(A). At this pressure, the theoretical temperature for melamine condensation (deposition) was calculated to be 316°C, leaving a margin of 20°C. The operating temperature of hot gas cooler 4 was set at 336°C. The hot process gas at the outlet of hot gas cooler 4 further passed through hot gas filter 5 to reduce the pressure to 3.2 bar(G) before entering crystallizer 6. Simultaneously, cold process gas was sent to crystallizer 6 by cold gas blower 9, with a mass ratio of 1.93 of the hot process gas to the cold process gas. After mixing in crystallizer 6, the temperature of the hot and cold process gases became 216°C, and the pressure dropped to 3.17 bar(G), causing melamine to crystallize into solid.

The pressure of the process gas at the outlet of the separator 7 was reduced to 3 bar(G), and the pressure of the process gas after passing through the urea scrubber 8 was reduced to 2.93 bar(G).

The power of the cold gas blower was 703 kw, and the power of the carrier gas compressor was 1,449 kw. According to the formula: energy consumption per unit product = (power of cold gas blower + power of carrier gas compressor) / melamine production, the energy consumption per unit product in this comparative example was calculated to be 344 kwh/t melamine.

The crystallizer in this comparative example has no discharge. The pipeline from the crystallizer to the separator contained 6.25 t/h of melamine solids and 297.5 t/h of process gas, with a gas-solid ratio of 48. The solids were not easily blown away by the gas flow, resulting in a short blockage period.

The melamine solids in this comparative example were all discharged from the bottom of the separator, transported using 5 t/h air, and the outside of the conveying pipe was heat-traced with a steam jacket. The internal pressure of the conveying pipe was 0.5 bar(G), and the temperature at the end of the conveying pipe was 130°C. The melamine product was obtained through the packaging device. The pressure difference between the separator and the conveying pipe was not too large. Based on a gas leakage rate of 0.02% at the bottom of the separator, the leaked gas was 0.0595 t/h. At 130°C, this leaked gas will not turn into a solid, which does not affect the purity of the product, which was 99.5%. However, the product temperature was high during packaging, which was prone to damage the packaging bags.

The obtained product was a single product without particle grading. The average particle diameter D50 of the product was 30 microns to 40 microns, and the bulk density was 0.45 g/cm³ to 0.5 g/cm³. The particle diameter distribution was relatively wide, barely suitable for producing melamine-formaldehyde resin glue, but the performance of the produced glue was unstable.

### Comparative Example 2: annual production capacity of 100,000 tons/year (12.5 t/h)

It should be noted that this comparative example was only based on a hypothetical 100,000-ton process flow and parameters, and does not represent the existence of corresponding process technology in reality.

170 t/h of hot process gas was pressurized to 15 bar(G) by the carrier gas compressor 1 and heated to 410°C by the carrier gas preheater 2 before entering the melamine reactor 3 as fluidizing carrier gas. 37.5 t/h of raw material urea was fed into the melamine reactor 3 (the mass ratio of carrier gas to urea was 4.53). At the outlet of the melamine reactor 3, 207.5 t/h of hot process gas was obtained, with a temperature of 410°C and a pressure of 12.5 bar(G).

The hot process gas at the outlet of melamine reactor 3 contained 12.5 t/h of melamine, equivalent to a mole fraction of 1.31%. The total pressure was 12.5 bar(G), and the partial pressure was calculated as 0.177 bar(A). At this pressure, the theoretical temperature for melamine condensation (deposition) was calculated to be 345°C, leaving a margin of 20°C. The operating temperature of hot gas cooler 4 was set at 365°C. The hot process gas at the outlet of hot gas cooler 4 further passed through hot gas filter 5 to reduce the pressure to 12.2 bar(G) before entering crystallizer 6. Simultaneously, cold process gas was fed into crystallizer 6 by cold gas blower 9, with a mass ratio of 3.13 of the cold process gas to hot process gas. After mixing in crystallizer 6, the temperature of the hot and cold process gases became 209°C, and the pressure dropped to 12.05 bar(G), resulting in the crystallization of melamine into solid.

The pressure of process gas at the outlet of separator 7 was reduced to 11.67 bar(G), and the pressure of process gas after passing through urea scrubber 8 was reduced to 11.5 bar(G).

The power of the cold gas blower was 1,870 kw, and the power of the carrier gas compressor was 1,690 kw. According to the formula: energy consumption per unit product = (power of cold gas blower + power of carrier gas compressor) / melamine production, the energy consumption per unit product in this comparative example was calculated to be 285 kwh/t melamine.

The crystallizer in this comparative example has no discharge. The pipeline from the crystallizer to the separator contains 12.5 t/h of melamine solids and 845 t/h of process gas, with a gas-solid ratio of 68. The solids were not easily blown away by the gas flow, resulting in a short blockage period.

All the melamine solids in this comparative example were discharged from the bottom of the separator, transported using 10 t/h of air, with natural heat dissipation outside the conveying pipe. The pressure inside the conveying pipe was 0.5 bar(G), and the temperature at the end of the conveying pipe was 55°C. The melamine product was obtained through the packaging device. Due to the too large pressure difference between the conveying pipe and the separator, the leakage rate at the bottom of the separator was calculated as 0.1%, resulting in a leakage of 0.845 t/h of gas. At 55°C, 30% of this leaked gas turned into solid ammonium carbamate, which mixed with the product, reducing the purity of the product by 2%, i.e., reaching 97.5%.

The obtained product was a single product without particle grading. The average particle diameter D50 of the product was 30 microns to 40 microns, and the bulk density was 0.45 g/cm³ to 0.5 g/cm³. The particle diameter distribution was relatively wide, barely suitable for producing melamine-formaldehyde resin glue, but the performance of the produced glue was unstable.

Based on the above comparison, it can be seen that the unit energy consumption in Examples 1-3 was relatively low; the gas-solid ratio in the pipeline from the crystallizer to the separator was high, making it less prone to blockage; the product was graded into two specifications based on particle size; the amount of leaked process gas was relatively small, and it will not affect the purity of the product, nor will it damage the packaging bag due to heat.

Obviously, the above examples are merely examples provided for clear illustration, and are not limitations on the implementation modes. For those skilled ordinary in the art, other variations or modifications in different forms can be made based on the above description. It is neither necessary nor possible to exhaust all implementation modes here. The obvious variations or modifications derived from this are still within the scope of protection of the present invention.

## Claims

1. A process for producing melamine by gas-phase quenching, comprising the following steps:
cooling hot process gas from an outlet of a melamine reactor in a hot gas cooler and filtering it in a hot gas filter successively, then admitting the cooled and filtered gas into a crystallizer where it directly contacts cold process gas, thereby producing solid A and mixed gas, discharging the solid A from the bottom of the crystallizer, and feeding the mixed gas into a separator to perform gas-solid separation and thereby obtaining solid B;
wherein the solid A is transported into a high-pressure collecting device A using high-pressure conveying gas A, and a temperature of the high-pressure conveying gas A is ensured to be 120°C or above during transportation; after performing gas-solid separation in the high-pressure collecting device A, the solid A is transported into a packaging device A using low-pressure conveying gas A, and a temperature of the solid A at the end of transportation pipe is ensured not to exceed 85°C; and a pressure of the high-pressure conveying gas A is in a range from 2 barG to 8 barG and is lower than a pressure inside the crystallizer, and a pressure of the low-pressure conveying gas A is in a range from 0.2 barG to 6.5 barG and is lower than a pressure of the high-pressure conveying gas A.

2. The process for producing melamine by gas-phase quenching according to claim 1, wherein the solid B is transported into a high-pressure collecting device B using high-pressure conveying gas B, and a temperature of the high-pressure conveying gas B is ensured to be 120°C or above during transportation; after performing gas-solid separation in the high-pressure collecting device B, the solid B is transported into a packaging device B using low-pressure conveying gas B, and a temperature of solid B at the end of transportation pipe is ensured not to exceed 85°C; and a pressure of the high-pressure conveying gas B is in a range from 2 barG to 8 barG and is lower than a pressure inside the separator, and a pressure of the low-pressure conveying gas B is in a range from 0.2 barG to 6.5 barG and is lower than a pressure of the high-pressure conveying gas B.

3. The process for producing melamine by gas-phase quenching according to claim 1 or 2, wherein a mass ratio of the cold process gas to the hot process gas which enter the crystallizer is in a range from 2.6 to 3.2.

4. The process for producing melamine by gas-phase quenching according to claim 1 or 2, wherein a pressure inside the crystallizer is in a range from 4 barG to 9.8 barG.

5. The process for producing melamine by gas-phase quenching according to claim 1 or 2, wherein a discharge amount from the bottom of the crystallizer accounts for 5% to 50% of the total amount of solids produced.

6. The process for producing melamine by gas-phase quenching according to claim 1 or 2, wherein a mass ratio of carrier gas to urea which enter the melamine reactor is in a range from 3.1 to 4.5.

7. The process for producing melamine by gas-phase quenching according to claim 1 or 2, wherein the top of the melamine reactor has a temperature ranging from 380°C to 405°C, and the top of the melamine reactor has a pressure ranging from 5 barG to 10 barG.

8. An apparatus for producing melamine by gas-phase quenching, comprising:
a crystallizer discharging unit, comprising a crystallizer discharging device, a high-pressure collecting device A and a packaging device A, wherein an inlet of the crystallizer discharging device is connected to the bottom of the crystallizer, and an outlet of the crystallizer discharging device is connected to an inlet of the high-pressure collecting device A through a high-pressure conveying pipe A; a lower outlet of the high-pressure collecting device A is connected to the packaging device A through a low-pressure conveying pipe A; an outer wall of the high-pressure conveying pipe A is equipped with a heat preservation structure to ensure that a temperature inside the high-pressure conveying pipe A is 120°C or above, and a pressure inside the high-pressure conveying pipe A is in a range from 2 barG to 8 barG and is lower than a pressure inside the crystallizer; an outer wall of the low-pressure conveying pipe A is equipped with a cooling structure to ensure that a temperature inside the end of the low-pressure conveying pipe A does not exceed 85°C, and a pressure of the low-pressure conveying pipe A is in a range from 0.2 barG to 6.5 barG and is lower than the pressure inside the high-pressure conveying pipe A; and
a separator discharging unit, comprising a separator discharging device and a packaging device B, wherein an inlet of the separator discharging device is connected to the bottom of the separator, and an outlet of the separator discharging device is connected to the packaging device B, and the inlet of the separator is connected to an upper outlet of the crystallizer.

9. The apparatus for producing melamine by gas-phase quenching according to claim 8, wherein the crystallizer discharging device is a screw conveyor;
and/or, the outlet of the separator discharging device is connected to the inlet of the high-pressure collecting device B through the high-pressure conveying pipe B, and a lower outlet of the high-pressure collecting device B is connected to the packaging device B through a low-pressure conveying pipe B; and
an outer wall of the high-pressure conveying pipe B is equipped with a heat preservation structure to ensure that a temperature inside the high-pressure conveying pipe B is 120°C or above, and a pressure inside the high-pressure conveying pipe B is in a range from 2 barG to 8 barG and is lower than the pressure inside the separator; and an outer wall of the low-pressure conveying pipe B is equipped with a cooling structure to ensure that a temperature inside the end of the low-pressure conveying pipe B does not exceed 85°C, and a pressure inside the low-pressure conveying pipe B is in a range from 0.2 barG to 6.5 barG and is lower than the pressure inside the high-pressure conveying pipe B.

10. The apparatus for producing melamine by a gas-phase quenching according to claim 8 or 9, wherein the apparatus further comprises a carrier gas compressor, a carrier gas preheater, a melamine reactor, a hot gas cooler, a crystallizer, a separator, a urea scrubber and a cold gas blower which are sequentially connected;
an upper outlet of the separator is connected to the urea scrubber, to cool the gas to produce recyclable cold process gas; and
a cold gas outlet of the urea scrubber is connected to an inlet of the cold gas blower, and an outlet of the cold gas blower is connected to a cold process gas inlet of the crystallizer.
